# EUROPÄISCHE PATENTSCHRIFT

(11) **EP 0 696 604 B1**
(45) Veröffentlichungstag und Bekanntmachung des Hinweises auf die Patenterteilung: **27.01.1999**
(21) Anmeldenummer: 95111995.7
(22) Anmeldetag: 31.07.1995
(51) Int. Cl.: C08G 18/12

(54) **Cycloaliphatische thermoplastische Polyurethanelastomere**
Cycloaliphatic thermoplastic polyurethane elastomers
Elastomères de polyuréthane thermoplastiques cycloaliphatiques

(30) Priorität: 11.08.1994 DE 4428458
(43) Veröffentlichungstag der Anmeldung: 14.02.1996
(73) Patentinhaber: BAYER AG, 51368 Leverkusen (DE)
(72) Erfinder: Pudleiner, Heinz, Dr., D-47800 Krefeld (DE); Dhein, Rolf, Dr., D-47800 Krefeld (DE); Hugl, Herbert, Dr., D-51467 Bergisch Gladbach (DE); Müller, Hanns-Peter, Dr., D-51519 Odenthal (DE); Heidingsfeld, Herbert, D-50226 Frechen (DE); Hoppe, Hans-Georg, D-42799 Leichlingen (DE)

(56) Entgegenhaltungen:
- EP-A- 0 154 768
- EP-A- 0 282 771
- EP-A- 0 604 890
- US-A- 3 849 360
- CHEMICAL ABSTRACTS, vol. 101, no. 26, Dezember 1984, Columbus, Ohio, US; abstract no. 232028x, 'POLYURETHANE SOLUTIONS' Seite 72 ; & JP-A-59 108 021 (SANYO)
- JOURNAL OF APPLIED POLYMER SCIENCE, Bd.51, Nr.1, 3. Januar 1994, NEW YORK, US Seiten 43 - 49 AHN ET AL 'THE PROPERTIES OF POLYURETHANES WITH MIXED CHAIN EXTENDERS AND MIXED SOFT SEGMENTS'

## Beschreibung

Polyurethan-Elastomere werden aus drei Basiskomponenten hergestellt: einem Polyisocyanat, einem Makrodiol und einem Kettenverlängerer, der in der Regel ein niedermolekulares Diol, Diamin, Aminoalkohol oder Wasser ist. Wird als Kettenverlängerer ein Diol verwendet, enthält das gebildete Polyurethan abgesehen von durch Nebenreaktionen gebildeten Allophanat- oder Biuret-Gruppen ausschließlich Urethanbindungen. Werden Wasser, Aminoalkohole oder Diamine verwendet, entstehen sowohl Urethan- als auch Harnstoff-Gruppen und das Produkt wird als Polyurethan-Harnstoff bezeichnet.

Polyurethan-Elastomere sind (AB)ₙ-Blockcopolymere und bilden wegen der mehr oder minder stark ausgeprägten Unverträglichkeit der Polymerblöcke Mikrodomänen aus, die als Hart- und Weichsegment bezeichnet werden. Das Hartsegment (Block A) wird von den Polymerbereichen gebildet, die durch Reaktion der Isocyanatkomponente mit dem Kettenverlängerer entstehen, zeigt im allgemeinen einen hohen Grad an Kristallinität und ist hauptsächlich für die mechanischen und thermischen Eigenschaften, d.h. das Aufschmelzverhalten bei der Umformung, verantwortlich. Die Schmelztemperaturen des Hartsegments müssen oberhalb der Gebrauchstemparatur liegen. Das Weichsegment (Block B) wird aus den Polyether- oder Polyesteranteilen der Polymerkette gebildet und ist in der Regel amorph oder nur wenig kristallin; die Glastemperatur muß weit unter der Gebrauchstemperatur liegen.

Ihren thermoplastischen Charakter erhalten die Polyurethan-Elastomere dadurch, daß die Hartsegmente bei hohen Temperaturen aufschmelzen, so daß eine Abnahme der Viskosität und Bildung einer Schmelze erfolgt. Bei Abkühlung, z.B. im Wasserbad hinter einem Extruder, kristallisieren die Hartsegmente und es entsteht der feste elastomere Formkörper.

Gegenstand der Erfindung sind Polyurethanelastomere erhältlich aus
A) 5-Isocyanato-1-(isocyanatomethyl)-1.3.3-trimethylcyclohexan (Isophorondiisocyanat) und/oder Bis-(4-isocyanatocyclohexyl)-methan und
B) Polytetrahydrofuran und/oder Hexandiolpolycarbonat mit Molekulargewichten von 1500 bis 3000
im Molverhältnis A) : B) = 1,2 : 1 bis 30 : 1,
C) einem Kettenverlängerergemisch aus
C.1) 5-Amino-3-aminomethyl-1,3,3-trimethyl-cyclohexan (Isophorondiamin) und/oder Bis-(4-Amino-cyclohexyl)-methan und
C.2) 1,6-Hexandiol, 1,4-Butandiol, Diethylenglykol, Di- und Tripropylenglykol und/oder Hydrochinon-di-β-hydroxyethylether,
im Molverhältnis C.1) : C.2) = 60 : 40 bis 20 : 80 und
D) 0,01 bis 7 Äquivalent-% bezogen auf NCO-Gruppen in A) eines Monoisocyanats, Monoalkohols oder Monoamins (als Molekulargewichtsregler)
wobei A), B) und C.2) in einer ersten Stufe zu einem NCO-Präpolymer und dieses mit C.1 und D) in einer zweiten Stufe zum Polyurethan-Harnstoff umgesetzt werden, und wobei das Molverhältnis von NCO-Gruppen zu allen aktiven Wasserstoffatomen 0,9:1 bis 1,2:1 ist.

Ein weiterer Gegenstand der Erfindung sind Formkörper, insbesondere Folien, Schläuche (für medizinische Zwecke) und Katheter aus diesen Polyurethanelastomeren bzw. die Verwendung der Polyurethanelastomeren zur Herstellung dieser Formkörper.

Diol-verlängerte Polyurethane für den Einsatz als Schlauch- und Kathetermaterial sind unter den Bezeichnungen Pellethane® (Dow Chemical Co.) und Tecoflex® (Thermedics Inc.) bekannt. Diese Produkte werden in der Regel nach dem "One-shot"-Verfahren, bei dem alle Komponenten in der Schmelze vermischt werden und dann miteinander reagieren, oder nach dem zweistufigen "Prepolymer"-Verfahren, bei dem aus dem Makrodiol und der Isocyanatkomponente zunächst ein Voraddukt gebildet wird, das in einer zweiten Stufe mit dem Verlängerer zum Polyurethan umgesetzt wird, hergestellt. Diese Produkte zeigen zwar schon gute Biokompatibilität, bedürfen jedoch speziell bei der Reaktion der Hydroxylgruppen mit den Isocyanatgruppen des Zusatzes eines Schwermetall-Katalysators, z.B. Dibutylzinndilaurat. Diese Katalysatoren sind inhärent zelltoxisch und sollten in Materialien für den Einsatz im Körper möglichst nicht vorhanden sein.

Besonders die Umsetzung cycloaliphatischer Polyisocyanate mit Glykolen verläuft selbst bei Temperaturen zwischen 150°C und 180°C verhältnismäßig langsam, so daß zur Erhöhung der Reaktionsgeschwindigkeit auf eine Katalyse, in der Regel mit Schwermetallverbindungen, nicht verzichtet werden kann. Bei den weicheren (Shore A 70 bis 85) Produkteinstellungen der auf dem Bis-(4-isocyanatcyclohexyl)-methan-Isomerengemisch basierenden aliphatischen thermoplastischen Polyurethanen tritt zudem bei der Verarbeitung das Problem auf, daß die hergestellten Formteile, besonders direkt nach Verarbeitung, noch eine große Oberflächenklebrigkeit aufweisen. Diese unerwünschte Eigenschaft erfordert spezielle Verarbeitungstechniken, bei denen die einzelnen gefertigten Teile sich wenigstens solange nicht berühren, bis dieser Effekt durch die vervollständigte Kristallisation der Hartsegmente abgeklungen ist.

Demgegenüber bietet die Reaktion von Aminen mit Isocyanaten gewisse Vorteile: Sie benötigt in der Regel keine externe Katalyse; die Reaktion von primären, aliphatischen Aminen mit Isocyanaten läuft sogar schon bei Raumtemperatur so schnell ab, daß eine homogene Reaktionsdurchführung in der Schmelze wegen der sofortigen Ausfällung von Harnstoff nicht mehr möglich ist. Die gebildeten Harnstoff-Gruppen bilden besser kristallisierende Hartsegmente, so daß mit einer Oberflächenklebrigkeit nicht zu rechnen ist.

Polyurethan-Harnstoffe an sich sind schon lange bekannt. Hochmolekulare, elastomere Polyurethan-Harnstoffe aus Polyisocyanaten, Makrodiolen und Polyaminen konnten jedoch nach den oben beschriebenen üblichen Verfahren nicht unverdünnt hergestellt werden, da die Reaktion zwischen aliphatischen Amino- und den Isocyanatgruppen so energisch abläuft, daß vor der Verfestigung des Reaktionsproduktes keine homogene Mischung der Reaktionspartner mehr möglich ist. Deshalb werden derartige Polyharnstoffe bisher immer in stark verdünnter Lösung hergestellt. Das bedeutet, daß entweder viel Lösungsmittel von der Herstellung mittransportiert werden muß, ein zusätzlicher, technisch aufwendiger Eindampfschritt der Herstellung des Polyurethan-Harnstoffs nachgeschaltet werden muß und bei der Beschichtung eines medizinischen Artikels, wie z.B. eines Katheters, besonders auf die lösungsmittelfreie Trocknung des Auftrags geachtet werden muß.

Für den medizinischen Einsatz wird die Lösung eines aromatischen Polyurethan-Harnstoffs unter dem Namen Biomer® (Ethicon Corp.) angeboten.

Bei solchen normalen Polyurethan-Harnstoffen liegt in der Regel der Schmelzbereich oberhalb der Zersetzungstemperatur, so daß solche Produkte für eine thermoplastische Verarbeitung ungeeignet sind.

Die US-Patentschrift 4 062 834 beansprucht ein Verfahren zur Herstellung eines rein aromatischen thermoplastischen Polyetherurethan-Harnstoffs, bei dem das Polymer in Lösung mit Wasser als Kettenverlängerer hergestellt wird und anschließend verfahrenstechnisch aufwendig ausgefällt und aufgearbeitet werden muß. Anhand der aufgeführten mechanischen Daten wird deutlich, daß die nach diesem Verfahren hergestellten Produkte gegenüber z.B. rein Diamin-kettenverlängerten Polyurethan-Harnstoffen deutlich niedrigere Zugfestigkeiten aufweisen. Anhand der dort beschriebenen Molekulargewichte wird verständlich, daß die Thermoplastizität auf Kosten eines hohen Molekulargewichtes erzielt wurde.

In der DE-OS 24 23 764 wird ein Verfahren zur Herstellung von Polyurethan-Harnstoffen offengelegt, das die Umsetzung von Polyisocyanaten, Makrodiolen und Polyaminen in der Schmelze nach dem Reaktionsextrusionsverfahren beansprucht. Zwar wird in dieser Anmeldung auch die thermoplastische Verarbeitbarkeit der so hergestellten Produkte erwähnt, jedoch erreichen diese Polymere längst nicht das ausgezeichnete, mit üblichen Polyurethanen vergleichbare Aufschmelz- und Verarbeitungsverhalten der erfindungsgemäßen Polyurethan-Harnstoffe. Darüber hinaus wird die Verwendung solcher Produkte für Katheter- und Schlauchanwendungen nicht erwähnt.

In der EP 0 396 270 werden aus der Schmelze verarbeitbare Polyurethan-Harnstoff-Copolymere und ein Verfahren zu ihrer Herstellung beansprucht. Erfindungswesentlich ist hierbei u.a. auch die Einstellung eines NCO/OH bzw. NH-Verhältnisses von vorzugsweise 120, was allerdings, wie auch unsere Vergleichsversuche belegen, zu Polyurethan-Harnstoffen mit im Vergleich zu Polyurethanen deutlich schlechterem Aufschmelzverhalten führt. Darüber hinaus erklärt sich hieraus auch die immer notwendige Nachhärtung ("post cure") für die nach allen Verfahrensvarianten der EP 0 396 270 hergestellten Polyurethan-Harnstoffe.

Die erfindungsgemäßen Polyurethanelastomere weisen demgegenüber die folgenden Vorteile auf:

Melt-Volume-Index bei 190°C/5 kg von 1 bis 200 cm³/10 min, Transparenz, bessere Zellverträglichkeit, Schmelzviskositäten und Fließeigenschaften wie reine thermoplastische Polyurethane, Verarbeitbarkeit in Standard-Maschinen ohne Verklebungen der Produkte, Herstellbarkeit auf Reaktionsextrudern, bessere Extrusionsqualitäten, Herstellung ohne Lösungsmittel und ohne Katalysator.

Zur Herstellung der erfindungsgemäßen Polyurethan-Elastomere wird als Komponente A) 5-Isocyanat-1-(isocyanat-methyl)-1,1,3-trimethyl-cyclohexan (Isophorondiisocyanat) (bevorzugt ca. 70 Gew.-% cis Isomer, ca. 30 Gew.-% trans Isomer) und/oder 4,4'-Bis-(isocyanatcyclohexyl)methan, bevorzugt mit einem trans,trans-Isomer-Gehalt von 20 bis 95 Gew.-% verwendet.

Als höhermolekulare Polyhydroxyverbindung B) muß Polytetrahydrofuran (Polytetrahydrofurandiol) oder Hexandiolpolycarbonat mit Molekulargewicht (Zahlenmittel) von 1500 bis 3000 eingesetzt werden. Es hat sich als besonders vorteilhaft erwiesen, als Komponente B) vorzugsweise Polytetrahydrofuran mit einem mittleren Molekulargewicht (Zahlenmittel) von ca. 1750 bis 2500 zu verwenden.

Polytetrahydrofuran mit mittlerem Molekulargewicht 1000 ergab dagegen klebrige, nicht weiterverarbeitbare Produkte.

Als Komponente B) können auch Mischungen von 99 bis 1 Gew.-% Polytetramethylenglykolether und 1 bis 99 Gew.-% Hexandiolpolycarbonat verwendet werden. Letztere beeinflussen im allgemeinen jedoch die Klebrigkeit ungünstig, so daß diese Verfahrensform nicht bevorzugt wird.

Das Molverhältnis von Komponente A) zu Komponente B) soll 1,2:1 bis 30:1 bevorzugt 1,5:1 bis 20:1 betragen. Besonders bevorzugt sind Verhältnisse von 2:1 bis 12:1. Produkte dieser Art ergeben Elastomere mit Härten von 80 Shore A bis 80 Shore D, bevorzugt sind solche von 85 Shore A bis 70 Shore D.

Als Kettenverlängerungsgemische C) können solche mit cycloaliphatischen Diaminen, als Komponente C.1) bevorzugt Isophorondiamin oder Bis-(4-aminocyclohexyl)-methan und einem oder mehreren (z.B. zwei oder drei) weiteren Diolen C.2) mit Molekulargewicht 62 bis 399, vorzugsweise 1,4-Butandiol, 1,6-Hexandiol, Diethylenglykol, Dipropylenglykol, Tripropylenglykol und Hydrochinon-di-β-hydroxyethylether, besonders bevorzugt 1,6-Hexandiol verwendet werden. Bevorzugt wird ein Gemisch von 60 bis 20 mol-% Isophorondiamin und 40 bis 80 mol-% 1,6-Hexandiol; besonders bevorzugt ist das Molverhältnis 50 bis 30 mol-% bis 50 bis 70 mol-%.

Ferner können in dem Fachmann bekannter Form monofunktionelle Verbindungen D) (Molekulargewichtsregler) mitverwendet werden. Beispiele sind Monoalkohole wie Butanol, Ethylhexanol, Isobutylalkohol, 1-Octanol, Stearylalkohol oder Monoamine wie Dibutylamin, N-Methylstearylamin, Piperidin oder Monoisocyanate wie Stearylisocyanat, bevorzugt Dibutylamin.

Die Komponente D) muß in Mengen von 0,01 bis 7 Äquivalent-%, bevorzugt 2 bis 6 Äquivalent-%, bezogen auf den NCO-Gruppengehalt der Komponente A), eingesetzt werden. Die optimalen Verarbeitungseigenschaften werden nur dann bei den Polymeren festgestellt, wenn der für Kettenregler vergleichsweise hohe Gehalt von 2 bis 6 Äquivalent-%, bezogen auf Komponente A), eingesetzt wird. Darüber hinaus wurde überraschenderweise gefunden, daß bei trotz des vergleichsweise hohen Gehalts an Molekulargewichtsregler klebfreie und gut weiterverarbeitbare Produkte mit ausgezeichneten mechanischen und thermischen Eigenschaften erhalten werden.

Als Komponente E) können - bevorzugt 0,1 bis 3 Gew.-% (bezogen auf die Gesamtmenge aller Komponenten) - an Wachsen, Antioxidantien und/oder UV-Absorbern miteingesetzt werden. Bevorzugt werden Mischungen von Stabilisatoren eingesetzt. Es sollen - wenn überhaupt- nur die minimal notwendigen Mengen von solchen Additiven eingesetzt werden, so daß die Zellverträglichkeit nicht beeinträchtigt wird.

Als Antioxidantien können alle hierfür bekannten Produkte, wie sie z.B. in der EP-A 12 343 beschrieben sind, eingesetzt werden. Bevorzugt sind Antioxidantien auf der Basis sterisch gehinderter Phenole, wie z.B. 2,6-Di-t-butyl-4-methyl-phenol und Pentaerythrityl-tetrakis-3-(3,5-di-t-butyl-4-hydroxyphenyl)-propionat (Irganox® 1010, Ciba Geigy).

Bei der Herstellung der Polyurethan-Elastomere können die üblichen Katalysatoren, Trennmittel, Antistatika, Flammschutzmittel, Füllstoffe und Einfärbmittel (siehe z.B. DE-OS 28 54 409, DE-OS 29 20 501 und DE-P 33 29 775) zugegeben werden. Bevorzugt wird jedoch auf die oben genannten Additive verzichtet, insbesondere, wenn die Elastomere für medizinische Zwecke verwendet werden sollen; ausgenommen sind spezielle Füllstoffe, wie z.B. Bariumsulfat oder Wismutoxid zur Herstellung röntgenkontrastfähiger Mischungen, und Trennmittel, zur Erzielung besonders glatter Oberflächen.

Als Katalysatoren können z.B. tertiäre Amine, organische Metallverbindungen, insbesondere organische Zinn-, Blei-, und Titanverbindungen eingesetzt werden, z.B. Zinn-II-acetat, Zinn-II-ethylhexanoat, Dibutylzinndilaurat oder Bleiacetat. Bevorzugt wird ohne Katalysator gearbeitet.

Als Trennmittel können Wachse oder Öle verwendet werden, ferner z.B. langkettige Verbindungen mit Carboxyl-, Ester-, Amid-, Urethan- oder Harnstoff-gruppen, wie sie beispielsweise in der DE-OS 2 204 270 aufgeführt sind. Bevorzugt wird Bisethylenstearylamid.

Die Mengen an Reaktionskomponenten A) bis C) werden für die erfindungsgemäßen Polyurethan-Elastomeren so gewählt, daß das NCO/OH- bzw. NCO/NHR-Verhältnis von Diisocyanat zu OH-Verbindungen bzw. Aminverbindungen zwischen 0,9 und 1,2, bevorzugt zwischen 0,98 und 1,05 liegt.

Die erfindungsgemäßen Polyurethan-Harnstoffe werden bevorzugt nach einem Zweistufenprozeß kontinuierlich oder batchweise hergestellt, wobei besonders bevorzugt die Komponenten A) und B) sowie die aliphatische Dihydroxyverbindung der Komponente C) zu einem NCO-haltigem Prepolymer umgesetzt werden und dann das Prepolymer in einem geeigneten organischen Lösungsmittel, z.B. Toluol gelöst, und mit dem z.B. in Toluol/Isopropanol gelösten cycloaliphatischen Diamin der Komponente C) kettenverlängert wird. Bevorzugt wird jedoch ein Kettenverlängerungsverfahren, bei dem das Prepolymer als Schmelze, die monofunktionelle Verbindung (Komponente D) und das cycloaliphatische Diamin der Komponente C) in die Reaktionsschnecke dosiert werden und hier zu dem fertigen thermoplastischen Polyurethan-Elastomer ausreagieren. Im Gegensatz zur EP-A 0 396 270 wird bei diesem Herstellungsverfahren das fertig ausreagierte Polyurethan-Elastomer erhalten, das nicht mehr, wie in EP-A 0396 270 beschrieben, in einem weiteren Verfahrensschritt durch Lagern an feuchter oder normaler Raumluft nachgehärtet werden muß.

Die erfindungsgemäßen Polyurethan-Elastomere verfügen über eine sehr gute Zellverträglichkeit, wie aus den Daten der Tabelle 1 entnommen werden kann. Vorteilhaft wirkt sich hier die katalysatorfreie Herstellbarkeit der Polyurethane aus.

Bei den Zellverträglichkeitsuntersuchungen wurden L-Zellen angezüchtet und anschließend in Gegenwart einer zu untersuchenden Probe bzw. deren wäßrigen Extrakts weitergezüchtet. Von den nach Versuchsende ausgewerteten verschiedenen Parametern zeigten die Auswertung der DNS- und syntheseleistung besonders anschaulich die Vorteile der Polyurethan-Harnstoffproben.

### Bestimmungsmethoden

**Tabelle 1:**

| Zellverträglichkeit von Polyurethan-Harnstoff aus Beispiel 2 mit Vergleichsproben | | | | |
|---|---|---|---|---|
| | Vergleichsbeispiel 1 | Vergleichsbeispiel 2 | Vergleichsbeispiel 3 | Polyurethan-Harnstoff (Beispiel 2) |
| DNS-Synthese-Ausbeute relativ zur Kontrolle | 35% | 50% | 62% | 61% |
| Proteinsynthese-Ausbeute unter dem Einfluß des wäßrigen Extrakts rel. zur Kontrolle | 65% | 70% | 82% | 78% |
| Vergleichsbeispiel 1: Handelsübliches aliphatisches thermoplastisches Polyurethan für medizinische Anwendungen der Härte 85 Shore A | | | | |
| Vergleichsbeispiel 2: Handelsübliches aliphatisches thermoplastisches Polyurethan für medizinische Anwendungen der Härte 93 Shore A | | | | |
| Vergleichsbeispiel 3: Handelsübliches aliphatisches thermoplastisches Polyurethan für medizinische Anwendungen der Härte 60 Shore D | | | | |
| Beispiel 2: Polyetherurethan-Harnstoff (Siehe Beispiele) (92 Shore A) | | | | |

Bei diesen Untersuchungen dürfen nur Materialien gleicher Härteeinstellung miteinander verglichen werden. Die Tabelle 1 zeigt, daß der Polyetherurethan-Harnstoff deutlich besser als das vergleichbar harte Material abschneidet und fast so gut wie das wesentlich härtere Polyurethan ist.

### Zeitabhängige Oberflächenklebrigkeitstests

Mehrere flächengleiche Proben des zu untersuchenden Materials werden jeweils 10 Minuten bei 150°C temperiert. Alle Proben werden gleichzeitig aus dem Temperierofen herausgenommen (Meßzeitpunkt t=0) und bis zur Messung bei Raumtemperatur aufbewahrt. In einer Meßapparatur werden nun bei gleichem Anpreßdruck und Anpreßzeit zwei Prüfflächen aus gleichem Material aneinander gepreßt und bei gleicher Abzuggeschwindigkeit die Kraft zum Trennen der Proben bestimmt. Die Messungen erfolgen jeweils an Proben nach 2, 3, 4, 5 und 6 Minuten nach Temperierung.

**Tabelle 2:**

| Oberflächenklebrigkeit [in N/mm] | | | | | |
|---|---|---|---|---|---|
| | 2 min. | 3 min. | 4 min. | 5 min. | 6 min. |
| Vergleichsbeispiel 3 | 0,90 | 0,60 | 0,5 | 0,2 | 0,1 |
| Vergleichsbeispiel 1 | 0,95 | 0,95 | 0,9 | 0,85 | 0,4 |
| Beispiel 2 | 0,2 | 0,2 | 0,15 | 0,12 | 0,1 |
| Beispiel 8 | 0,5 | 0,45 | 0,35 | 0,25 | 0,2 |

Beim Produkt aus Beispiel 2 klingt die Oberflächenklebrigkeit deutlich früher ab als bei den Vergleichsbeispielen. Darüber hinaus wird belegt, daß das Produkt mit dem Polytetrahydrofuran (PTHF)-Weichsegment mit Molekulargewicht 2000 weniger klebrig ist als das mit dem 1000-er PTHF.

Ein großer Vorteil der erfindungsgemäßen thermoplastischen Polyurethan-Harnstoffe ist darin zu sehen, daß sie ausgezeichnete mechanische, optische und toxikologische Eigenschaften mit der Möglichkeit der klebfreien thermoplastischen Verarbeitbarkeit durch Spritzguß oder Extrusion verbinden. Wie Tabelle 3 zeigt, verhalten sich die erfindungsgemäßen Polyurethane in ihrem temperaturabhängigem Schmelzflußindex (MVI = Melt-Volume-Index) wie harnstoffgruppen-freie thermoplastische Polyurethane.

**Tabelle 3:**

| Melt-Volume Indices | | |
|---|---|---|
| Melt-Volume-Index (MVI) | | |
| | MVI (190°C/5 kg)/cm³/10min. | MVI (200°C/5 kg)/cm³/10min. |
| Beispiel 1 | 32,14 | 65,03 |
| Beispiel 2 | 2,98 | 8,4 |
| Vergleichsbeispiel 4 | 4,46 | 12,59 |
| Vergleichsbeispiel 1 | 29,29 | 126,28 |
| Vergleichsbeispiel 5 | 0,06 | 0,44 |
| Vergleichsbeispiel 6 | 0,01 | 1,22 |
| Vergleichsbeispiel 7 | 0,2 | 1,4 |
| Beispiel 3 | 4,41 | 18,95 |
| Beispiel 4 | 152,6 | 392 |
| Beispiel 5 | 11,45 | 29,32 |
| Beispiel 6 | 2,37 | 6,5 |
| Beispiel 7 | 1,01 | 3,37 |

### Meßbedingungen: DIN ISO 1133

Auch in Praxis erweisen auch die erfindungsgemäßen Polyurethan-Harnstoff als gut verarbeitbar. Transparente, stippenfreie Katheterschläuche und Blasfolienschläuche konnten in einem stabilen Extrusionsprozeß hergestellt werden.

Aus der Tabelle 4 ist ersichtlich, daß das Aufschmelzen des Polyetherurethan-Harnstoffs aus Beispiel 2 für Katheter auf einem Brabender-Meßextruder PL 2000 bei konstanter Temperatur (198°C) und Drehzahl (19 U/min.) ein gleichmäßiges Drehmoment und einen gleichbleibenden Schmelzedruck über die Meßzeit erzeugt. Diese Daten belegen, daß der Polyurethan-Harnstoff für einem stabilen Verarbeitungsprozeß geeignet ist, und unerwünschte, der eigentlichen Polymerbildungsreaktion nachgeschaltete zusätzliche Härtungsreaktionen auf der Verarbeitungsmaschinen nicht stattfinden.

**Tabelle 4:**

| Zeitlicher Verlauf ausgewählter Extrusionsprozeßdaten von Beispiel 2 | | |
|---|---|---|
| Zeit/min | Drehmoment/NM | Druck/bar |
| 0 | 119,5 | 48 |
| 1 | 124,7 | 51 |
| 2 | 124,8 | 52 |
| 3 | 125,3 | 52 |
| 4 | 125,8 | 52 |
| 5 | 125,8 | 51 |
| 6 | 125,5 | 52 |
| 7 | 125 | 52 |
| 8 | 125,8 | 52 |
| 9 | 126,4 | 52 |
| 10 | 122,9 | 51 |
| 11 | 123,7 | 52 |
| 12 | 122,8 | 51 |
| 13 | 124,4 | 51 |
| 14 | 125,7 | 50 |
| 15 | 122,8 | 50 |
| 16 | 124,9 | 50 |
| 17 | 124,7 | 50 |
| 18 | 124,4 | 50 |
| 19 | 121,7 | 50 |
| 20 | 123,7 | 51 |

### Beispiele:

### Beispiel 1:

In einem Planschliffkolben mit Rührer und Innenthermometer werden 720 Teile Polytetrahydrofuran (Terathane 2000®) und 76,6 Teile 1,6-Hexandiol 1 Stunde bei 120°C im Wasserstrahlvakuum entwässert (Wassergehalt < 0,04 Gew.%). Es werden 329,8 Teile 5-Isocyanat-1-(isocyanat-methyl)-1,1,3-trimethyl-cyclohexan (Isophorondiisocyanat) zugegeben und 3 Stunden bei 120°C bis zum Erreichen der theoretischen NCO-Zahl von 3,55 Gew. (gemessen 3,5 Gew.-%) gerührt.

Anschließend werden 12 Teile Ethylenbisstearylamid und 15,3 Teile Di-n-butylamin zugegeben. Dieses Voraddukt wird in 634 Teilen Toluol gelöst und unter Rühren bei Raumtemperatur zu einer Lösung von 73,6 Teilen 5-Amino-3-aminomethyl-1,3,3-trimethyl-cyclohexan in 2990 Teilen Toluol/Isopropanol (70:30) zugetropft. Man erhält eine farblose, transparente und homogene Lösung des Polyetherurethan-Harnstoffs, die nach Trocknung einen transparenten, klebfreien und farblosen Film ergab. Nach Spritzguß der gehäckselten Filme wurde an den Spritzgußprüfkörpern eine Zugfestigkeit von 45,5 MPa und eine Reißdehnung von 560% gemessen.

### Beispiel 2:

### Dieses Beispiel zeigt bevorzugte Herstellung der Polyurethan-Harnstoffe:

Aus 587 Teilen wasserfreiem Polytetrahydrofuran (Terathane 2000®) vom durchschnittlichen Molekulargewicht 2000, 62,5 Teilen 1,6-Hexandiol und 269 Teilen 1-Isocyanato-3-isocyanatomethyl-3,5,5-trimethylcyclohexan (Isophorondiisocyanat) wird unter Rühren bei 120°C unter Stickstoff im Verlauf von 3 Stunden ein 3,5 % freie Isocyanatgruppen enthaltendes Voraddukt hergestellt und mit 9,8 Teilen Ethylenbisstearylamid-Wachs versetzt. Dieses Voraddukt wurde bei 80°C unter Stickstoff gelagert.

Aus einem Kessel werden pro Minute 1700 Teile des bei 80°C unter Stickstoff gelagerten Produktes über eine Zahnradpumpe, aus einem zweiten bei 80°C gehaltenen Kessel 110 Teile 5-Amino-3-aminomethyl-1,3,3-trimethyl-cyclohexan pro Minute und aus einem dritten bei 30°C gehaltenen Kessel 11 Teile Di-n-butylamin pro Minute in den Einlaßstutzen der mit 200 U/min gleichsinnig drehenden Zweiwellenschneckenmaschine (zweiwellige gleichsinnig drehende, selbstreinigende Schneckenmaschine des Typs ZDSK 53 der Firma Werner & Pfleiderer) dosiert. Über die Maschinenlänge wurden ungefähre Produkttemperaturen von 145 - 235°C gemessen. Die niedrigsten Temperaturen werden dabei jeweils an oder kurz vor der Austrittsdüsenlochplatte festgestellt. Die aus der Maschine tretenden Produktstränge werden in einem Wasserbad gekühlt und in einem Granulator zerkleinert. Das Granulat ist klebfrei und völlig transparent und wird anschließend bei 70°C getrocknet. Nach Spritzguß wurde an den Spritzgußprüfkörpern eine Zugfestigkeit von 39,9 MPa und eine Reißdehnung von 556% gemessen.

### Beispiel 3:

Wie in Beispiel 1 beschrieben, wird aus 480 Teilen Polytetrahydrofuran (Terathane 2000®), 128,15 Teile 1,6-Hexandiol und 468,77 Teile 5-Isocyanat-1-(isocyanatmethyl)-1,1,3-trimethyl-cyclohexan (Isophorondiisocyanat) bei 120°C das Prepolymer hergestellt.

Anschließend werden 12 Teile Ethylenbisstearylamid und 21,8 Teile Di-n-butylamin zugegeben, dieses Voraddukt in 634 Teilen Toluol gelöst und unter Rühren bei Raumtemperatur zu einer Lösung von 123,08 Teilen 5-Amino-3-aminomethyl-1,3,3-trimethyl-cyclohexan in 2990 Teilen Toluol/Isopropanol (70:30) zugetropft. Man erhält eine farblose, transparente und homogene Lösung des Polyetherurethan-Harnstoffs, die nach Trocknung einen transparenten, klebfreien und farblosen Film ergab. Nach Spritzguß der gehäckselten Filme wurde an den Spritzgußprüfkörpern eine Zugfestigkeit von 34,5 MPa und eine Reißdehnung von 395 % gemessen.

### Beispiel 4:

Wie in Beispiel 1 beschrieben wird aus 780 Teilen Polytetrahydrofuran (Terathane 2000®), 63,74 Teile 1,6-Hexandiol und 295,04 Teile 5-Isocyanat-1-(isocyanatmethyl)-1,1,3-trimethyl-cyclohexan (Isophorondiisocyanat) bei 120°C das Prepolymer hergestellt.

Anschließend werden 12 Teile Ethylenbisstearylamid und 13,74 Teile Di-n-butylamin zugegeben, dieses Voraddukt in 634 Teilen Toluol gelöst und unter Rühren bei Raumtemperatur zu einer Lösung von 61,22 Teilen 5-Amino-3-aminomethyl-1,3,3-trimethyl-cyclohexan in 2990 Teilen Toluol/Isopropanol (70:30) zugetropft. Man erhält eine farblose, transparente und homogene Lösung des Polyetherurethan-Harnstoffs, die nach Trocknung einen transparenten, klebfreien und farblosen Film ergab. Nach Spritzguß der gehäckselten Filme wurde an den Spritzgußprüfkörpern eine Zugfestigkeit von 31,2 MPa und eine Reißdehnung von 650 % gemessen.

### Beispiel 5:

Wie in Beispiel 1 beschrieben wird aus 720 Teilen Hexandiolpolycarbonat (Desmophen 2020®), 76,93 Teilen 1,6-Hexandiol und 329,12 Teilen 5-Isocyanat-1-(isocyanat-methyl)-1,1,3-trimethyl-cyclohexan (Isophorondiisocyanat) bei 120°C das Prepolymer hergestellt.

Anschließend werden 12 Teile Ethylenbisstearylamid und 15,42 Teile Di-n-butylamin zugegeben, dieses Voraddukt in 634 Teilen Toluol gelöst und unter Rühren bei Raumtemperatur zu einer Lösung von 72,49 Teilen 5-Amino-3-aminomethyl-1,3,3-trimethyl-cyclohexan in 2990 Teilen Toluol/Isopropanol (70:30) zugetropft. Man erhält eine farblose, transparente und homogene Lösung des Polyetherurethan-Harnstoffs, die nach Trocknung einen transparenten, klebfreien und farblosen Film ergab. Nach Spritzguß der gehäckselten Filme wurde an den Spritzgußprüfkörpern eine Zugfestigkeit von 53,1 MPa und eine Reißdehnung von 440 % gemessen.

### Beispiel 6:

Wie in Beispiel 1 beschrieben wird aus 720 Teilen Hexandiolpolycarbonat (Desmophen 2020®), 66,67 Teilen 1,6-Hexandiol und 349,32 Teilen Bis-(4-isocyanat-cyclohexyl)-methan (Desmodur W®) bei 120°C das Prepolymer hergestellt.

Anschließend werden 12 Teile Ethylenbisstearylamid und 13,79 Teile Di-n-butylamin zugegeben, dieses Voraddukt in 634 Teilen Toluol gelöst und unter Rühren bei Raumtemperatur zu einer Lösung von 64,02 Teilen 5-Amino-3-aminomethyl-1,3,3-trimethyl-cyclohexan in 2990 Teilen Toluol/Isopropanol (70:30) zugetropft. Man erhält eine farblose, transparente und homogene Lösung des Polyetherurethan-Harnstoffs, die nach Trocknung einen transparenten, klebfreien und farblosen Film ergab. Nach Spritzguß der gehäckselten Filme wurde an den Spritzgußprüfkörpern eine Zugfestigkeit von 45,4 Mpa und eine Reißdehnung von 425 % gemessen.

### Beispiel 7:

Wie in Beispiel 1 beschrieben wird aus 720 Teilen Hexandiolpolycarbonat (Desmophen 2020®), 64,15 Teilen 1,6-Hexandiol und 339,75 Teilen Bis-(4-isocyanat-cyclohexyl)-methan (Desmodur W®) bei 120°C das Prepolymer hergestellt.

Anschließend werden 12 Teile Ethylenbisstearylamid und 13,41 Teile Di-n-butylamin zugegeben, dieses Voraddukt in 1000 Teilen Toluol gelöst und unter Rühren bei Raumtemperatur zu einer Lösung von 76,11 Teilen 4,4'-Bis(aminocyclohexyl)-methan in 2624 Teilen Toluol/Isopropanol (70:30) zugetropft. Man erhält eine farblose, transparente und homogene Lösung des Polyetherurethan-Harnstoffs, die nach Trocknung einen transparenten, klebfreien und farblosen Film ergab. Nach Spritzguß der gehäckselten Filme wurde an den Spritzgußprüfkörpern eine Zugfestigkeit von 45,8 MPa und eine Reißdehnung von 405 % gemessen.

### Beispiel 8 (nicht erfindungsgemäß):

Wie in Beispiel 1 beschrieben wird aus 720 Teilen Polytetrahydrofuran 1000 (Terathane 1000®), 60,76 Teile 1,6-Hexandiol und 360,88 Teile 5-Isocyanat-1-(isocyanat-methyl)-1,1,3-trimethyl-cyclohexan (Isophorondiisocyanat) bei 120°C das Prepolymer hergestellt.

Anschließend werden 12 Teile Ethylenbisstearylamid und 16,77 Teile Di-n-butylamin zugegeben, dieses Voraddukt in 634 Teilen Toluol gelöst und unter Rühren bei Raumtemperatur zu einer Lösung von 58,35 Teilen 5-Amino-3-aminomethyl-1,3,3-trimethyl-cyclohexan in 2990 Teilen Toluol/Isopropanol (70:30) zugetropft. Man erhält eine farblose, transparente und homogene Lösung des Polyetherurethan-Harnstoffs, die nach Trocknung einen transparenten, farblosen, allerdings stark klebenden Film ergab. Nach Spritzguß der gehäckselten Filme wurde an den Spritzgußprüfkörpern eine Zugfestigkeit von 45 MPa und eine Reißdehnung von 480 % gemessen.

### Vergleichsbeispiele:

### Vergleichsbeispiel 1:

Handelsübliches aliphatisches thermoplastisches Polyurethan für medizinische Anwendungen der Härte 85 Shore A

### Vergleichsbeispiel 2:

Handelsübliches aliphatisches thermoplastisches Polyurethan für medizinische Anwendungen der Härte 93 Shore A

### Vergleichsbeispiel 3:

Handelsübliches aliphatisches thermoplastisches Polyurethan für medizinische Anwendungen der Härte 60 Shore D

### Vergleichsbeispiel 4:

Handelsübliches aromatisches thermoplastisches Polyurethan für medizinische Anwendungen der Härte 85 Shore A

### Vergleichsbeispiel 5 (entsprechend EP 0 396 270, Weichsegment 70 Gew.-%):

350 Teile Polytetrahydrofuran, 12,48 Teile 1,4-Butandiol und 12,58 Teile 1,5-Diamino-2-methylpentan (Dytek A® von DuPont, Wilmington) werden bei Raumtemperatur gemischt. 124,8 Teilen bei 40°C geschmolzenes 4,4'-Bis-(isocyantophenyl)-methan (MDI) werden schnell zugegeben und zwei Minuten intensiv mit einem Rührer vermischt. Die Schmelze wird auf ein Teflonblech ausgegossen und 1 Stunde bei 125°C und anschließend in feuchter Luft nachgehärtet. Die Polymermatte wurde zerkleinert und vom Granulat der MVI gemessen (siehe Tabelle 3).

### Vergleichsbeispiel 6 (entsprechend EP 0 396 270, Weichsegment 60 Gew.-%)

360 Teile Polytetrahydrofuran, 23,5 Teile 1,4-Butandiol und 23,5 Teile 1,5-Diamino-2-methylpentan (Dytek A® von DuPont, Wilmington) werden bei Raumtemperatur gemischt. 193 Teilen bei 40°C geschmolzenes 4,4'-Bis(isocyantophenyl)-methan (MDI) werden schnell zugegeben und zwei Minuten intensiv mit einem Rührer vermischt. Die Schmelze wird auf ein Teflonblech ausgegossen und 1 Stunde bei 125°C und anschließend in feuchter Luft nachgehärtet. Die Polymermatte wurde zerkleinert und vom Granulat der MVI gemessen (siehe Tabelle 3).

### Vergleichsbeispiel 7 (entsprechend EP 0 348 105)

71,4 Teile Polytetrahydrofuran 1000 (Terathane 1000®, von der DuPont, Wilmington) und 214,3 Teile Polyethylenglykol 1000 werden gemischt und bei 120°C im Wasserstrahlvakuum entwässert. Unter Stickstoff und intensivem Rühren werden 117,9 Teile 4,4'-Bis-(isocyanatophenyl)-methan (MDI) zugegeben. Die Mischung wurde 1 bis 1,5 Stunden bei 60 bis 65°C gerührt. Anschließend wurde das Prepolymer in 857,6 Teilen N,N-Dimethylacetamid (DMAC) gelöst. 4,34 Teile Ethanolamin, 4,27 Teile Ethylendiamin und 0,82 Teile Dimethylethylendiamin wurden in 350 Teilen DMAC gelöst und schnell unter intensivem Rühren zu der Prepolymerlösung zugegeben. Die Reaktionslösung wurde 3 Stunden bei 85°C gerührt. Man erhielt eine schwach gelbe, viskose Lösung, die zu einem Film getrocknet wurde. Der getrocknete Film wurde zerkleinert und vom Granulat der MVI gemessen (siehe Tabelle 3).

## Patentansprüche

1.Polyurethanelastomer erhältlich aus
A) 5-Isocyanato-1-(isocyanatomethyl)-1.3.3-trimethylcyclohexan (Isophorondiisocyanat) und/oder Bis-(4-isocyanatocyclohexyl)-methan und
B) Polytetrahydrofuran und/oder Hexandiolpolycarbonat mit Molekulargewichten von 1500 bis 3000
im Molverhältnis A) : B) = 1,2 : 1 bis 30 : 1,
C) einem Kettenverlängerergemisch aus
C.1) 5-Amino-3-aminomethyl-1,3,3-trimethyl-cyclohexan (Isophorondiamin) und/oder Bis-(4-Amino-cyclohexyl)-methan und
C.2) 1,6-Hexandiol, 1,4-Butandiol, Diethylenglykol, Di- und Tripropylenglykol und/oder Hydrochinon-di-β-hydroxyethylether,
im Molverhältnis C.1) : C.2) = 60 : 40 bis 20 : 80 und
D) 0,01 bis 7 Äquivalent-% bezogen auf NCO-Gruppen in A) eines Monoisocyanats, Monoalkohols oder Monoamins (als Molekulargewichtsregler)
wobei A), B) und C.2) in einer ersten Stufe zu einem NCO-Präpolymer und dieses mit C.1 und D) in einer zweiten Stufe zum Polyurethan-Harnstoff umgesetzt werden, und wobei das Molverhältnis von NCO-Gruppen zu allen aktiven Wasserstoffatomen 0,9:1 bis 1,2:1 ist.

2. Formkörper aus den Polyurethanelastomeren des Anspruchs 1.

3. Folien aus den Polyurethanelastomeren des Anspruchs 1.

4. Medizinische Schläuche und Katheter aus den Polyurethanelastomeren des Anspruchs 1.

5. Verwendung der Polyurethanelastomere des Anspruchs 1 zur Herstellung von Formkörpern, insbesondere von Folien, medizinischen Schläuchen und Kathetern.

6. Verfahren zur Herstellung eines Polyurethan-Elastomers unter Einhaltung eines Molverhältnisses der NCO-Gruppen zu den gesamten aktiven Wasserstoffatomen von 0,9:1 bis 1,2:1 durch Umsetzung von (A), (B) und (C2) zu einem NCO-Präpolymer und Umsetzung des Präpolymers mit (C1) und (D), wobei
(A) 5-Isocyanato-1-(isocyanatomethyl)-1,3,3-trimethylcyclohexan und/oder Bis-(4-isocyanatocyclohexyl)-methan und
(B) Polytetrahydrofuran und/oder Hexandiolpolycarbonat mit mittleren Molekulargewicht 1500-3000 ist,
bei einem Molverhältnis von (A):(B) von 1,2:1 bis 30:1,
C.1) 5-Amino-3-aminomethyl-1,3,3-trimethyl-cyclohexan (Isophorondiamin) und/oder Bis-(4-Amino-cyclohexyl)-methan und
C.2) 1,6-Hexandiol, 1,4-Butandiol, Diethylenglykol, Di- und Tripropylenglykol und/oder Hydrochinon-di-β-hydroxyethylether,
im Molverhältnis C1:C2 = 60:40 bis 20:80 sind und
D) 0,01 bis 7 Äquivalent-% bezogen auf NCO-Gruppen in A) eines Monoisocyanats, Monoalkohols oder Monoamins (als Molekulargewichtsregler) ist.

## Claims

1. Polyurethane elastomers obtainable from
A) 5-isocyanato-1-(isocyanatomethyl)-1,3,3-trimethylcyclohexane (isophorone diisocyanate) and/or bis-(4-isocyanatocyclohexyl)methane, and
B) a polytetrahydrofuran and/or a hexanediol polycarbonate with molecular weights of 1500 to 3000
in a molar ratio A) : B) = 1.2 : 1 to 30 : 1,
C) a chain extender mixture comprising
C.1) 5-amino-3-aminomethyl-1,3,3-trimethylcyclohexane (isophoronediamine) and/or bis-(4-aminocyclohexyl)-methane and
C.2) 1,6-hexanediol, 1,4-butanediol, diethylene glycol, di- and tripropylene glycol and/or hydroquinone di-β-hydroxyethyl ether,
in a molar ratio C.1) : C.2) = 60 : 40 to 20 : 80, and
D) 0.01 to 7 equivalent %, based on the NCO groups in A), of a monoisocyanate, monoalcohol or monoamine (as a molecular weight regulator),
wherein A), B) and C.2) are reacted in a first stage to form an NCO prepolymer, and the latter is reacted with C.1 and D) in a second stage to form the polyurethane-urea, and wherein the molar ratio of NCO groups to all the active hydrogen atoms is 0.9:1 to 1.2:1.

2. Mouldings from the polyurethane elastomers of claim 1.

3. Films from the polyurethane elastomers of claim 1.

4. Medical flexible tubing and catheters from the polyurethane elastomers of claim 1.

5. The use of the polyurethane elastomers of claim 1 for the production of mouldings, particularly films, medical flexible tubing and catheters.

6. A process for producing a polyurethane elastomer, whilst maintaining a molar ratio of NCO groups to total active hydrogen atoms of 0.9:1 to 1.2:1, by the reaction of (A), (B) and (C2) to form an NCO prepolymer and reaction of the prepolymer with (C1) and (D), wherein
A) is 5-isocyanato-1-(isocyanatomethyl)-1,3,3-trimethylcyclohexane (isophorone diisocyanate) and/or bis-(4-isocyanatocyclohexyl)-methane, and
B) is a polytetrahydrofuran and/or a hexanediol polycarbonate with an average molecular weight of 1500 to 3000,
in a molar ratio of (A):(B) of 1.2:1 to 30:1,
C.1) is 5-amino-3-aminomethyl-1,3,3-trimethylcyclohexane (isophoronediamine) and/or bis-(4-aminocyclohexyl)-methane, and
C.2) is 1,6-hexanediol, 1,4-butanediol, diethylene glycol, di- and tripropylene glycol and/or hydroquinone di-β-hydroxyethyl ether,
in a molar ratio C1:C2 = 60:40 to 20:80, and
D) is 0.01 to 7 equivalent %, based on the NCO group in A), of a monoisocyanate, monoalcohol or monoamine (as a molecular weight regulator).

## Revendications

1. Elastomères de polyuréthane obtenables à partir de
A) 5-isocyanato-I-(isocyanatométhyl)-1,3,3-triméthylcyclohexane (isophoronediisocyanate) et/ou de bis-(4-isocyanatocyclohexyl)-méthane et de
B) polytétrahydrofurane et/ou d'hexanediolpolycarbonate, possédant un poids moléculaire de 1500 à 3000 dans un rapport molaire A):B) de 1,2:1 à 30:1,
C) d'un mélange de prolongateurs de chaîne constitué de
C.1) 5-amino-3-aminométhyl-1,3,3-triméthylcyclohexane (isophoronediamine) et/ou de bis-(4-amine-cyclohexyl)-méthane et de
C.2) 1,6 hexanediol, de 1,4-butanediol, de diéthylèneglycol, de di- et de tripropylèneglycol et/ou d'hydroquinone-di-β-hydroxyéthyléther.
dans un rapport molaire C.1):C.2) de 60:40 à 20:80 et
D) 0,01 à 7 équivalents % par rapport aux groupes NCO de A) d'un monoisocyanate, d'un mono-alcool ou d'une monoamine (comme régulateur de poids moléculaire)
A), B) et C.2) étant transformés dans une première étape en un prépolymère comportant des groupes NCO, lequel est converti dans une deuxième étape avec C.1 et D) en un polyuréthane-urée, les groupes NCO et tous les atomes d'hydrogène actifs étant entre eux dans un rapport molaire de 0,9:1 à 1,2:1.

2. Pièces moulées constituées des élastomères de polyuréthane de la revendication 1.

3. Feuilles constituées des élastomères de polyuréthane de la revendication 1.

4. Tuyaux souples médicaux et cathéters constitués des élastomères de polyuréthane de la revendication 1.

5. Utilisation des élastomères de polyuréthane de la revendication 1 pour la production de pièces moulées, en particulier de feuilles, de tuyaux souples médicaux et de cathéters.

6. Procédé de production d'un élastomère de polyuréthane, en respectant un rapport molaire entre les groupes NCO et la totalité des groupes d'hydrogène actifs de 0,9 à 1,2:1, par transformation de (A), (B) et (C.2) en un prépolymère comportant des groupes NCO et mise en réaction de ce dernier avec (C.1) et (D), auquel cas
A) est du 5-isocyanato-I-(isocyanatométhyl)-1,3,3-triméthylcyclohexane et/ou du bis-(4-isocyanatocyclohexyl)-méthane et
B) est du polytétrahydrofurane et/ou de l'hexanediolpolycarbonate, possédant un poids moléculaire de 1500 à 3000
dans un rapport molaire A):B) de 1,2:1 à 30:1,
C.1) est du 5-amino-3-aminométhyl-1,3,3-triméthyl-cyclohexane (isophoronediamine) et/ou du bis-(4-amino-cyclohexyl)-méthane et
C.2) est du 1,6 hexanediol, du 1,4-butanediol, de diéthylèneglycol, du di- et du tripropylèneglycol et/ou de l'hydroquinone-di-β-hydroxyéthyléther. dans un rapport molaire C.1):C.2) de 60:40 à 20:80 et
D) est 0,01 à 7 équivalents % par rapport aux groupes NCO de A) d'un monoisocyanate, d'un mono-alcool ou d'une monoamine (comme régulateur de poids moléculaire).
